# EUROPÄISCHE PATENTSCHRIFT

(11) **EP 3 178 450 B1**
(45) Veröffentlichungstag und Bekanntmachung des Hinweises auf die Patenterteilung: **25.07.2018**
(21) Anmeldenummer: 16203221.3
(22) Anmeldetag: 09.12.2016
(51) Int. Cl.: A61F 2/962, A61F 2/24

(54) **EINFÜHRKATHETER UND KATHETERANORDNUNG**
INSERTION CATHETER AND CATHETER ASSEMBLY
CATHÉTER DE LARGAGE ET SYSTÈME DE CATHÉTER

(30) Priorität: 10.12.2015 DE 102015121501
(43) Veröffentlichungstag der Anmeldung: 14.06.2017
(73) Patentinhaber: Biotronik AG, 8180 Bülach (CH)
(72) Erfinder: Fargahi, Amir, 8180 Bülach (CH)
(74) Vertreter: Galander, Marcus

(56) Entgegenhaltungen:
- WO-A1-2014/109048
- US-A1- 2011 098 804
- US-A1- 2011 264 203
- US-A1- 2014 257 460

## Beschreibung

### Gebiet der Erfindung

Die Erfindung betrifft einen Einführkatheter zum Implantieren eines kardiovaskulären Implantats, welches ein selbstexpandierendes Grundgerüst aufweist, mit einem ersten, inneren Katheterschaft, welcher innerhalb von mindestens einem zweiten, äußeren Katheterschaft angeordnet ist, wobei das Implantat im distalen Bereich des Einführkatheters auf dem ersten, inneren Katheterschaft angeordnet ist und wobei ein Abschnitt nahe dem distalen Ende des zweiten, äußeren Katheterschafts als Implantatkapsel zur Umhüllung des Implantats während des Einführens ausgebildet ist. Sie betrifft des Weiteren eine mit diesem gebildete Katheteranordnung. Die Erfindung eignet sich insbesondere für Herzklappenprothesen, die eine Herzklappenanordnung aufweisen, welche an einem selbstexpandierenden Grundgerüst (Herzklappenstent) befestigt ist

Minimal invasive chirurgische Eingriffe gewinnen seit Jahren ständig an Bedeutung und sind beispielsweise zur Behandlung von Stenosen unverzichtbar. In letzter Zeit werden sie auch verstärkt bei der Implantation künstlicher Herzklappen eingesetzt. Geeignete Einführkatheter sind in großer konstruktiver Vielfalt bekannt und Gegenstand ständiger Weiterentwicklung. In den letzten Jahren wurde hier bei der Weiterentwicklung Kathetern besonderes Augenmerk geschenkt, die nicht nur das Setzen, sondern auch das Zurückziehen bzw. eine Positionierung von kardiovaskulären Implantaten ermöglichen.

Derartige Einführkatheter bestehen im Wesentlichen aus einem ersten, inneren Katheterschaft, auf dessen distalem Ende das Implantat angeordnet ist. Das Implantat und der erste, innere Katheterschaft sind von einem zweiten, äußeren Katheterschaft umgeben.

Der distale Bereich des zweiten äußeren Katheterschafts, der das Implantat umgibt, wird häufig als Implantatkapsel, oder gelegentlich als Katheterhülle bezeichnet. Die Implantatkapsel kann dabei aus dem gleichen Material wie der zweite äußere Katheterschaft oder aus einem anderen Material bestehen, welches mit dem zweiten äußeren Katheterschaft verbunden wird. Die Implantatkapsel ist in ihrer axialen Ausdehnung mindestens so lang wie das Implantat selbst, zumeist jedoch in ihrer axialen Ausdehnung etwas länger als das Implantat. Hier wird unter der Positionsbezeichnung "proximal" ein näher zum Operateur liegender Teil des Einführkatheters bezeichnet. "Distal" bezeichnet entsprechend einen Teil des Einführkatheters, der sich weiter entfernt vom Operateur befindet.

Häufig sind die Implantate unter Einsatz von Formgedächtnismaterial konstruiert. In diesen Fällen werden die Implantate während des Einführens an den Implantationsort durch die sie umgebende Katheterhülle in ihrer komprimierten Form gehalten. Durch Verschieben der Katheterhülle, beispielsweise nach proximal, verschwindet die Rückhaltekraft, die die Katheterhülle auf das Implantat ausübt, und das Implantat expandiert.

Vor kurzem wurden Lösungen vorgeschlagen, die ein Zurückführen ("resheathing") eines bereits freigesetzten Implantats in den Einführkatheter, also speziell in die Implantatkapsel, ermöglichen sollen. Derartige Lösungen werden von den Operateuren stark begrüßt, weil sie Korrekturen während des Einführvorganges ermöglichen und somit helfen, den Implantationsvorgang mit dem bestmöglichen Ergebnis abzuschließen. Speziell beim Zurückziehen von Herzklappenstents in die Implantatkapsel bzw., präziser formuliert, beim Wiederaufstülpen des distalen Katheterendes über den Stent, treten relativ hohe Reaktionskräfte auf. Diese führen zu komplexen Problemen wie beispielsweise in der Beschreibung des Standes der Technik in der US 2011/0098804 beschrieben und mit dem Versuch einer (teilweisen) Lösung verknüpft.

Speziell kann es, wie die Erfinder bei entsprechenden praktischen Erprobungen bekannter Systeme festgestellt haben, zu einem funktionellen Versagen der Implantatkapsel beim Zurückführen über das Implantat kommen, und zwar speziell zur Bildung von Knickstellen an der Implantatkapsel, die das Resheathen erschweren oder praktisch unmöglich machen. Derartige Probleme treten insbesondere bei Herzklappenprothesen auf, die eine Herzklappenanordnung aufweisen, welche an einem selbstexpandierenden Grundgerüst (Herzklappenstent) befestigt ist.

Der Erfindung liegt daher die Aufgabe der Bereitstellung eines verbesserten Einführkatheters für eine Herzklappenprothese, aufweisend eine Herzklappenanordnung befestigt in einem selbstexpandierenden Herzklappenstent, zugrunde, bei dem insbesondere eine Knickbildung im Bereich der Implantatkapsel weitgehend verhindert und hierdurch ein zuverlässiges und leichtes Resheathen ermöglicht werden soll.

Diese Aufgabe wird durch einen Einführkatheter mit den Merkmalen des Anspruchs 1 gelöst. Zweckmäßige Fortbildungen des Erfindungsgedankens sind Gegenstand der abhängigen Ansprüche. Mit der Erfindung wird weiterhin eine verbesserte Einführkatheteranordnung bereitgestellt.

Neben der oben erwähnten Erkenntnis der Erfinder schließt die vorliegende Erfindung den Gedanken ein, im Bereich der Implantatkapsel eine aus geometrisch unterschiedlichen Strukturelementen kombinierte Versteifung vorzusehen, die - anders als früher vorgeschlagene Lösungen - nicht einfach eine Gesamt-Versteifung der Kapsel durch Erhöhung der Wandstärke oder durch Vorsehen eines besonders dicken inneren Katheterschaftes darstellt. Diese früher vorgeschlagenen Lösungen haben nämlich nach den Feststellungen der Erfinder erhebliche Nachteile, speziell beim Einsatz in feineren Gefäßen bzw. mit aufwändigen Implantatkonstruktionen.

Spezieller gehört zur Erfindung der Gedanke, die Wandung der Implantatkapsel mit spiralig bzw. schraubenförmig gewickelten Drähten zu verstärken, die eine solche Querschnittsgestalt haben und derart miteinander zum Zusammenwirken gebracht werden, dass die erforderliche Biegsamkeit des Einführkatheters im Bereich der Implantatkapsel erhalten bleibt, eine Knickbildung jedoch weitgehend ausgeschlossen ist. Gemäß einer weiteren Überlegung der Erfinder ist hierzu eine Doppelhelix-Struktur aus einem Draht mit kreisförmigem (oder allgemeiner auch elliptischem) Querschnitt und einem Draht mit dreieckigem (oder allgemeiner auch annähernd dreieckigem) Querschnitt geeignet.
Diese geometrische Kombination ergibt eine gegenseitige Abstützung der beiden Helices bei Biegung in einer Weise, dass Biegungen mit sehr engem Radius - also einem beginnenden Abknicken - ein stark ansteigender Widerstand entgegengesetzt wird. Neben der hohen Wirksamkeit hat die vorgeschlagene Lösung die Vorteile einer relativ leichten Realisierbarkeit und geringen Materialaufwandes und somit relativ günstiger Gestehungskosten.

Die vorangehend beschriebenen geometrischen Strukturelemente zur Versteifung erstrecken sich mindestens über einen axialen Abschnitt der Implantatkapsel, bevorzugt über die gesamte axiale Länge der Implantatkapsel.

In einer Ausführung der Erfindung sind der erste und zweite Federdraht so ausgewählt, dass die Querschnittsflächen in einem Verhältnis zwischen 0,5 und 2, insbesondere zwischen 0,75 und 1,25, zueinander stehen. In einer weiteren Ausführung haben der erste und zweite Federdraht einen Durchmesser bzw. eine Kantenlänge des Dreiecks im Bereich zwischen 0,02 und 0,12 mm, insbesondere zwischen 0,04 und 0,06 mm. Versuche unter der Verwendung eines zweiten Federdrahtes mit einer Kantenlänge des Dreiecks von 0,1 mm haben ebenfalls vorteilhafte Ergebnisse gezeigt.

In weiteren Ausführungen der Erfindung sind der erste und/oder zweite Federdraht aus Materialien wie Edelstahl, Rostfreier Stahl 304V0, Federlegierungen auf Co-Cr Basis oder ähnlichen dem Fachmann bekannten Metallen wie Nitinol gebildet, wobei aus derzeitiger Sicht die Verwendung des gleichen Materials für beide Federdrähte vorteilhaft sein kann. Jedoch können grundsätzlich auch Drähte aus verschiedenen Metallen bzw. Legierungen miteinander kombiniert werden, wobei eine solche Kombination zusätzlich eine vorteilhafte Steuerung des Biegeverhaltens der Implantatkapsel ermöglichen kann.

In einer weiteren Ausführung der Erfindung ist in der Wandung der Implantatkapsel auf der Innenseite der ersten und zweiten Helix ein reibungsarmer Polymerschlauch angeordnet, der insbesondere Teflon oder HDPE oder dergleichen aufweist. Es versteht sich, dass neben den genannten Polymeren auch andere zum Einsatz kommen können, die hinsichtlich ihrer Einsetzbarkeit als Implantatmaterialien geprüft und geeignet sind und hinreichend gleitfähig bzw. reibungsarm gegenüber dem Implantat einerseits und des Innenkatheters andererseits sind.

Aus derzeitiger Sicht ist es bevorzugt, dass der Polymerschlauch eine Wanddicke im Bereich zwischen 0,02 und 0,1 mm, insbesondere zwischen 0,04 und 0,06 mm, aufweist.

In einer weiteren Ausführung ist vorgesehen, dass die Außenseite der Wandung der Implantatkapsel durch eine Polymerhülle gebildet ist, die insbesondere das Material PEBAX®, insbesondere 7033 oder 6333, oder dergleichen aufweist. Auch hier ist wiederum der Einsatz zahlreicher anderer Materialien möglich, die von medizinischen Produkten und speziell als Implantatmaterialien bekannt sind, etwa verschiedene Silikonmaterialien.

Die Implantatkapsel umgibt als distaler Bereich des zweiten, äußeren Katheterschaftes die Herzklappenprothese. Als Polymerhülle werden zweckmäßigerweise die vorangehend beschriebenen Materialien verwendet. Der zweite äußere Katheterschaft ist vorteilhafterweise als Polymerschaft ausgeführt, wobei jedoch nicht notwendigerweise das gleiche Polymer wie für die Polymerhülle verwendet werden muss. In einer Ausgestaltung der Erfindung kann auch der zweite, äußere Schaft (analog zur Implantatkapsel) bis zum als Implantatkapsel ausgebildeten distalen Bereich aus einem inneren, reibungsarmen Polymerschlauch und einer äußeren Polymerhülle bestehen, die einen stabilen Kern, beispielsweise aus geschnittenen bzw. geschlitzten Edelstahl, umschließen. Eine derartige Ausgestaltung zeichnet sich durch eine sehr gute axiale Schubfähigkeit aus, ohne ihre Flexibilität zu verlieren. Die Polymere der Implantatkapsel und der zweite äußere Schaft werden bevorzugt mittels Schweißen miteinander verbunden, wobei jedoch auch andere Befestigungsarten wie Kleben eine Alternative sein können.

In einer besonders bevorzugten Ausgestaltung der Erfindung wird der innere reibungsarme Polymerschlauch der Implantatkapsel mit einem inneren, reibungsarmen Polymerschlauch des zweiten äußeren Katheterschaftes verbunden, bevorzugt verschweißt. Die äußere Polymerhülle der Implantatkapsel wird mit der äußeren Polymerhülle des zweiten äußeren Schaftes verbunden, bevorzugt verklebt. Eine solche Ausgestaltung bietet für Herzklappenprothesen mit selbstexpandierenden Herzklappenstents die optimale Mischung aus Schubfähigkeit und Flexibilität. Die hier erreichte Schubfähigkeit erlaubt insbesondere ein problemloses Wiedereinfangen ("Resheating") einer teilweise freigesetzten Herzklappenprothese, welche einen selbstexpandierenden Herzklappenstent aufweist.

In einer besonders bevorzugten Variante der vorangehend geschilderten Ausgestaltung sind die Implantatkapsel und der zweite, äußere Schaft über einen Verbinder miteinander verbunden. Dieser Verbinder ist an seiner Innenseite mit dem geschnittenen Edelstahlkern und an seiner Außenseite mit dem inneren Polymerschlauch der Implantatkapsel verbunden, bevorzugt verklebt. Bevorzugt weist der Verbinder eine Stufe auf, um zu verhindern, dass das proximale Ende der Implantatkapsel scharfkantig absteht. Der Edelstahlkern wird bevorzugt nach dem Verbinden noch mit einem Schrumpfschlauch (Ethylen-Copolymer) umhüllt.

Für wichtige Anwendungen ist es bevorzugt, dass die Implantatkapsel (9) einen Innendurchmesser im Bereich zwischen 4,5 und 6,5 mm, insbesondere von 5,5 mm, und einen hierzu korrespondierenden Außendurchmesser im Bereich zwischen 5,0 und 7,0 mm, insbesondere von 6,0 mm, hat. Jedoch lässt sich die Erfindung auch bei Einführkathetern mit anderem Einsatzzweck als dem des Einführens von Stents oder Herzklappen und entsprechend anderem Innen- und/oder Außendurchmesser vorteilhaft einsetzen.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische Gesamtansicht eines Einführkatheters,
- Fig. 2: eine schematische Längsschnittdarstellung einer Implantatkapsel als Abschnitt des äußeren Katheterschafts eines Einführkatheters gemäß einer Ausführungsform der Erfindung,
- Fig. 3: die Verstärkungsstruktur der Implantatkapsel gemäß Fig. 1, und
- Fig. 4A und 4B: schematische Darstellungen zur Erläuterung der Wirkungsweise der Verstärkungsstruktur nach Fig. 3.

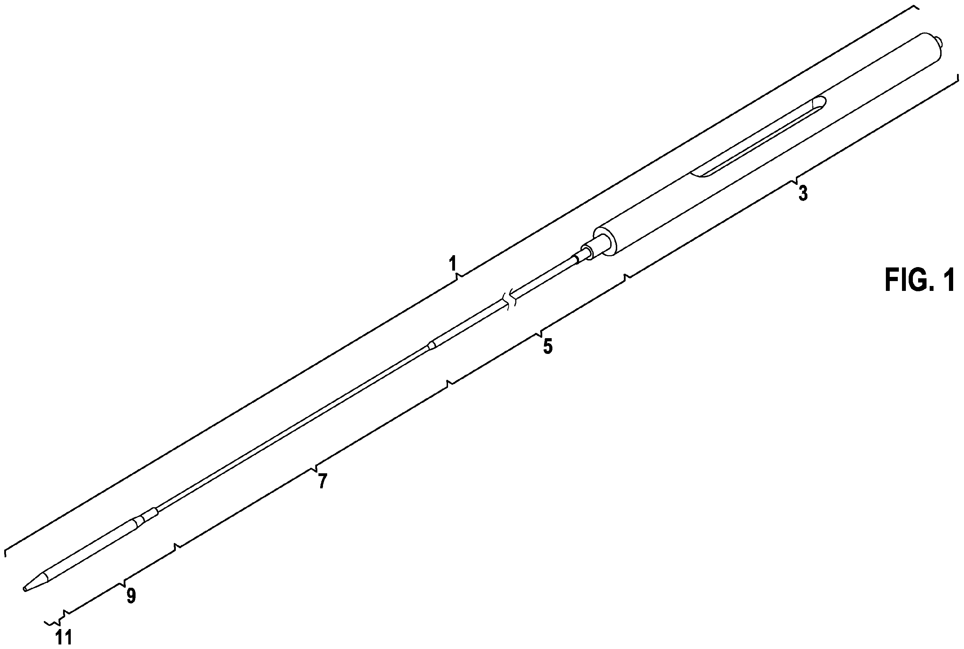

Fig. 1 zeigt in einer Gesamtansicht den Aufbau eines Einführkatheters 1, welches - vom proximalen Ende beginnend - einen Griffabschnitt 3, einen Stabilisatorabschnitt 5, einen Außenkatheterabschnitt 7, eine Implantatkapsel 9 und eine Katheterspitze 11 umfasst. In der Implantatkapsel ist (was in der Figur nicht zu erkennen ist) ein mittels des Einführkatheters 1 in ein Gefäß eines Patienten einzuführendes Implantat, ausgebildet als Herzklappenprothese mit einem selbstexpandierenden Herzklappenstent, untergebracht.

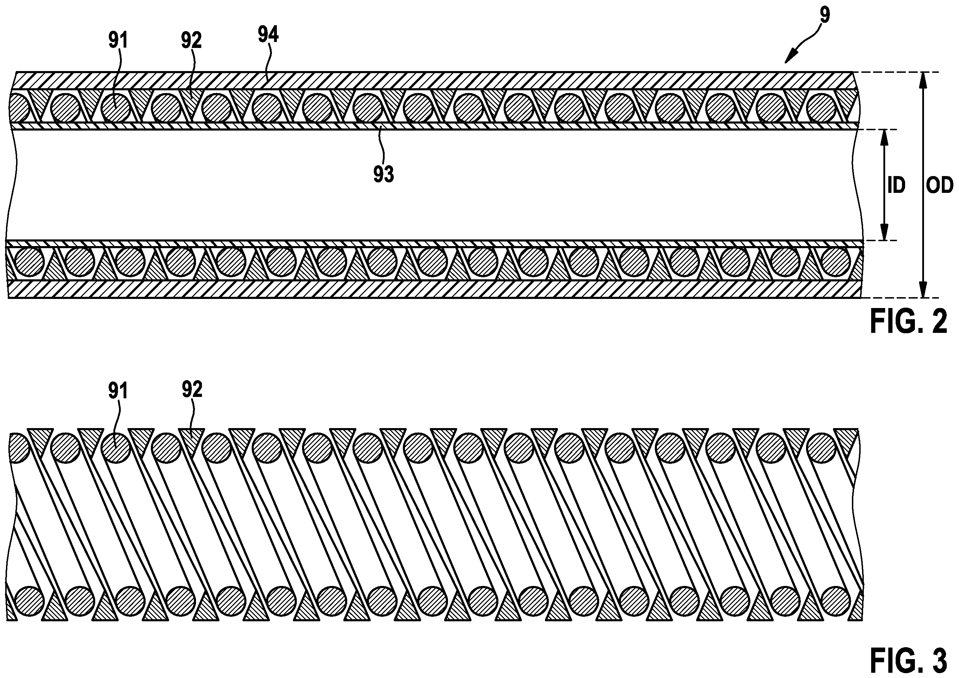

Fig. 2 zeigt schematisch den Aufbau einer Implantatkapsel 9 gemäß einer Ausführungsform der Erfindung. In der Wandung der Implantatkapsel 9 sind als Verstärkungsstruktur zwei ineinander gewickelte Drahtspiralen bzw. Helices 91, 92 vorgesehen, nämlich eine erste Helix 91 aus einem ersten Federdraht mit kreisförmigem Drahtquerschnitt und eine zweite Helix 92 aus einem zweiten Federdraht mit dreieckigem Querschnitt. Auf der Innenseite der beiden Helices 91, 92 ist ein reibungsarmer Polymerschlauch 93 angeordnet, und die Außenseite des Einführkatheters im Bereich der Implantatkapsel 9 ist durch eine Polymerhülle (Jacket) 94 gebildet.

Die hier gezeigte Orientierung der beiden Helices 91,92 ist nur beispielhaft. Die umgekehrte Orientierung, wo die Spitze des dreieckigen Querschnitts der zweiten Helix 92 zur abluminalen Seite (nach außen) zeigt, ist genauso vorteilhaft und je nach Fertigung gegebenenfalls zu bevorzugen.

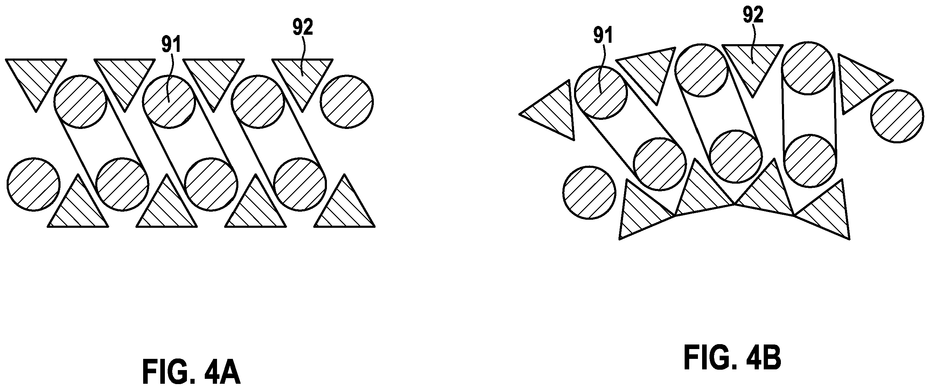

Fig. 3 zeigt die Verstärkungsstruktur aus den Helices 91, 92 nochmals separat, und Fig. 4B zeigt deren Biegeverhalten im Vergleich zu Fig. 4A (gestreckter Zustand ohne Biegemoment). Im Vergleich von Fig. 4A und 4B ist zu erkennen, dass die erste Helix 91 bei einer Biegung der Implantatkapsel durch die benachbart angeordneten ebenen Oberflächen der zweiten Helix 92 mit dreieckigem Querschnitt einer erheblichen Reibungskraft ausgesetzt wird, die bei zunehmendem Krümmungsradius einleuchtenderweise stark ansteigt, wodurch einer starken Biegung oder gar einem Knicken im Bereich der Implantatkapsel ein progressiv ansteigender Widerstand entgegengesetzt wird. Hingegen wirken die erwähnten Reibungskräfte zwischen erster und zweiter Helix sich bei einer Biegung mit einem relativ großen Krümmungsradius wenig aus, so dass die erwünschte Biegsamkeit des Einführkatheters auch im Bereich der Implantatkapsel jedenfalls für große Krümmungsradien weitgehend erhalten bleibt.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Einführkatheter (1) zum Implantieren eines kardiovaskulären Implantats, welches ein selbstexpandierendes Grundgerüst aufweist, mit einem ersten, inneren Katheterschaft, welcher innerhalb von mindestens einem zweiten, äußeren Katheterschaft (7) angeordnet ist, wobei das Implantat im distalen Bereich des Einführkatheters auf dem ersten, inneren Katheterschaft angeordnet ist und wobei ein Abschnitt nahe dem distalen Ende des zweiten, äußeren Katheterschafts als Implantatkapsel (9) zur Umhüllung des Implantats während des Einführens ausgebildet ist,
**dadurch gekennzeichnet, dass**
die Implantatkapsel eine Wandung aufweist, in der eine erste Helix (91) aus einem ersten Federdraht mit kreisförmigem Drahtquerschnitt und eine zweite Helix (92) aus einem zweiten Federdraht mit dreieckigem Querschnitt ineinander gewickelt sind.

2. Einführkatheter nach Anspruch 1, wobei die Querschnittsflächen des ersten und zweiten Federdrahts in einem Verhältnis zwischen 0,5 und 2, insbesondere zwischen 0,75 und 1,25, zueinander stehen.

3. Einführkatheter nach Anspruch 1 oder 2, wobei der erste und zweite Federdraht einen Durchmesser bzw. eine Kantenlänge des Dreiecks im Bereich zwischen 0,02 und 0,12 mm, insbesondere zwischen 0,04 und 0,06 mm, haben.

4. Einführkatheter nach einem der vorangehenden Ansprüche, wobei der erste und/oder zweite Federdraht aus Edelstahl, rostfreien Stahl oder Federlegierungen auf Co-Cr Basis gebildet sind.

5. Einführkatheter nach einem der vorangehenden Ansprüche, wobei in der Wandung der Implantatkapsel (9) auf der Innenseite der ersten und zweiten Helix ein reibungsarmer Polymerschlauch (93) angeordnet ist, der insbesondere Teflon oder HDPE oder dergleichen aufweist.

6. Einführkatheter nach Anspruch 5, wobei der Polymerschlauch (93) eine Wanddicke im Bereich zwischen 0,02 und 0,1 mm, insbesondere zwischen 0,04 und 0,06 mm, aufweist.

7. Einführkatheter nach einem der vorangehenden Ansprüche, wobei die Außenseite der Wandung der Implantatkapsel (9) durch eine Polymerhülle (94) gebildet ist, die insbesondere das Material Pebax 7033 oder dergleichen aufweist.

8. Einführkatheter nach Anspruch 7, wobei die Wandungsdicke der Polymerhülle (94) im Bereich zwischen 0,02 und 0,1 mm, insbesondere zwischen 0,04 und 0,06 mm, liegt.

9. Einführkatheter nach einem der vorangehenden Ansprüche, wobei die Implantatkapsel (9) einen Innendurchmesser im Bereich zwischen 4,5 und 6,5 mm, insbesondere von 5,5 mm, und einen hierzu korrespondierenden Außendurchmesser im Bereich zwischen 5,0 und 7,0 mm, insbesondere von 6,0 mm, hat.

## Claims

1. An insertion catheter (1) for implanting a cardiovascular implant, which insertion catheter comprises a self-expanding main structure, with a first, inner catheter shaft, which is arranged within at least one second, outer catheter shaft (7), wherein the implant is arranged on the first, inner catheter shaft in the distal region of the insertion catheter, and wherein a portion close to the distal end of the second, outer catheter shaft is formed as an implant capsule (9) for encasing the implant during the insertion,
**characterised in that**
the implant capsule has a wall, in which a first helix (91) made of a first spring wire with circular wire cross-section and a second helix (92) made of a second spring wire with triangular cross-section are wound in a manner intertwined with one another.

2. The insertion catheter according to claim 1, wherein the cross-sectional areas of the first and second spring wire are provided in a ratio to one another between 0.5 and 2, in particular between 0.75 and 1.25.

3. The insertion catheter according to claim 1 or 2, wherein the first and second spring wire have a diameter or an edge length of the triangle in the range between 0.02 and 0.12 mm, in particular between 0.04 and 0.06 mm.

4. The insertion catheter according to any one of the preceding claims, wherein the first and/or second spring wire are formed from high-grade steel, stainless steel, or Co-Cr-based spring alloys.

5. The insertion catheter according to any one of the preceding claims, wherein a low-friction polymer tube (93) is arranged in the wall of the implant capsule (9) on the inner side of the first and second helix and in particular comprises Teflon or HDPE or the like.

6. The insertion catheter according to claim 5, wherein the polymer tube (93) has a wall thickness in the range between 0.02 and 0.1 mm, in particular between 0.04 and 0.06 mm.

7. The insertion catheter according to any one of the preceding claims, wherein the outer side of the wall of the implant capsule (9) is formed by a polymer sleeve (94) which in particular comprises the material Pebax 7033 or the like.

8. The insertion catheter according to claim 7, wherein the wall thickness of the polymer sleeve (94) lies in the range between 0.02 and 0.1 mm, in particular between 0.04 and 0.06 mm.

9. The insertion catheter according to any one of the preceding claims, wherein the implant capsule (9) has an inner diameter in the range between 4.5 and 6.5 mm, in particular of 5.5 mm, and an outer diameter corresponding hereto in the range between 5.0 and 7.0, in particular of 6.0 mm.

## Revendications

1. Cathéter d'insertion (1) destiné à l'implantation d'un implant cardiovasculaire, lequel présente une armature de base auto-expansible, avec une première tige de cathéter intérieure, laquelle est disposée à l'intérieur d'au moins une deuxième tige de cathéter (7) extérieure, où l'implant est disposé sur la première tige de cathéter intérieure dans la région distale du cathéter d'insertion, et où une partie proche de l'extrémité distale de la deuxième tige de cathéter extérieure est conçue sous la forme d'une capsule d'implant (9) pour l'entourage de l'implant lors de l'insertion,
**caractérisé en ce que**
la capsule d'implant présente une paroi dans laquelle une première hélice (91) constituée d'un premier fil pour ressort avec une section transversale de fil en forme de cercle, et une deuxième hélice (92) constituée d'un deuxième fil pour ressort avec une section transversale triangulaire sont enroulées l'une dans l'autre.

2. Cathéter d'insertion selon la revendication 1, dans lequel les surfaces des sections transversales des premier et deuxième fils pour ressort sont dans un rapport entre eux entre 0,5 et 2, en particulier, entre 0,75 et 1,25.

3. Cathéter d'insertion selon la revendication 1 ou 2, dans lequel les premier et deuxième fils pour ressort ont un diamètre, respectivement, une longueur de côté du triangle, dans la plage entre 0,02 et 0,12 mm, en particulier, entre 0,04 et 0,06 mm.

4. Cathéter d'insertion selon l'une des revendications précédentes, dans lequel le premier et/ou le deuxième fil pour ressort est(sont) en acier spécial, en acier inoxydable ou dans des alliages pour ressorts à base de Co-Cr.

5. Cathéter d'insertion selon l'une des revendications précédentes, dans lequel un tuyau de polymère (93) à faible friction, qui présente en particulier du Téflon ou du PEHD ou un similaire, est disposé dans la paroi de la capsule d'implant (9) au niveau de la face intérieure des première et deuxième hélices.

6. Cathéter d'insertion selon la revendication 5, dans lequel le tuyau en polymère (93) présente une épaisseur de paroi dans la plage entre 0,02 et 0,1 mm, en particulier, entre 0,04 et 0,06 mm.

7. Cathéter d'insertion selon l'une des revendications précédentes, dans lequel la face extérieure de la paroi de la capsule d'implant (9) est formée par une enveloppe de polymère (94) qui présente en particulier la matière Pebax 7033 ou une similaire.

8. Cathéter d'insertion selon la revendication 7, dans lequel l'épaisseur de paroi de l'enveloppe de polymère (94) se situe dans la plage entre 0,02 et 0,1 mm, en particulier, entre 0,04 et 0,06 mm.

9. Cathéter d'insertion selon l'une des revendications précédentes, dans lequel la capsule d'implant (9) a un diamètre intérieur dans la plage entre 4,5 et 6,5 mm, en particulier de 5,5 mm, et un diamètre extérieur lui correspondant dans la plage entre 5,0 et 7,0 mm, en particulier, de 6,0 mm.
